# EUROPEAN PATENT APPLICATION

(11) **EP 2 703 496 A1**
(43) Date of publication of application: **05.03.2014**
(21) Application number: 12182613.5
(22) Date of filing: 31.08.2012
(51) Int. Cl.: C12Q 1/68, C12Q 1/70

(54) **Combination of PCR primers and a method for detection and identification of Human Papillomavirus (HPV)**

(71) Applicant: Innogenetics N.V., 9052 Zwijnaarde (BE)
(72) Inventor: Jannes, Geert, 9052 Zwijnaarde (BE); Mijs, Wouter, 9052 Zwijnaarde (BE)
(74) Representative: BiiP cvba

(57) **Abstract**

The present invention relates to the field of detection and genotyping of Human Papillomavirus (HPV) in clinical samples. More specifically, the present invention provides a combination of at least 11 SPF PCR primers for an unbiased and efficient detection of a broad selection of HPV genotypes, including all high-risk (HR) ones. The present invention further provides a method for HPV detection, using said PCR primer combination, that allows to select only HPV-positive samples for further steps of HPV identification.

## Description

### Field of the invention

The present invention relates to the field of detection and genotyping of Human Papillomavirus (HPV) in clinical samples. More specifically, the present invention provides a combination of PCR primers for an unbiased and efficient detection of a broad selection of HPV genotypes, including all high-risk (HR) ones. The present invention further provides a method for HPV detection, using said PCR primer combination, that allows to select only HPV-positive samples for further steps of HPV identification.

### Background of the invention

Human Papillomavirus (HPV) is an epitheliotropic pathogen that infects not only keratinocytes in the skin (cutaneous types) but also can target various mucosae such as the ones lining the respiratory and anogenital tract (mucosal types). Certain mucosal HPV types are confirmed to be responsible for many types of human malignancies, ranging from benign to carcinoma, mainly in cervical, vulvar, vaginal, anal, penile, head and neck tissues (Chaturvedi AK, et al., J Clin Oncol 2008;26:612-9; Chaturvedi AK, et al., J Clin Oncol 2011;29:4294-301) and recently even with a subset of colorectal cancers (Chen TH, et al., World J Gastroenterol. 2012 Aug 14;18(30):4051-8). In particular, cervical carcinoma, the third most prevalent cancer in women (Bosch FX, et al., J Clin Pathol. 2002; 55: 244-265) is unique in that the HPV DNA is found in virtually all tumour cases and in precursor lesions worldwide. In head and neck squamous cell carcinoma (HNSCC), the sixth most common cancer worldwide with an annual incidence of approximately 400000 and still rising, HPV infection is observed in about 30% of malignancies, preferentially in oropharynx region (Dufour X, et al., Eur Ann Otorhinolaryngol Head Neck Dis 2012;129:26-31; St Guily JL, et al., The EDiTH VI study. J Clin Virol 2011;51:100-4). The confirmed etiological role of HPV in malignant transformation has resulted in the development of molecular technologies focused on its detection (Bosch FX, et al., J Clin Pathol. 2002; 55: 244-265; Boulet GA, et al., Cancer Epidemiol Biomarkers Prev. 2008; 17: 810-17).

HPV is a circular double-stranded DNA (dsDNA) virus with genome of about 8000 base pairs. More than 130 HPV genotypes have been described based on minimum 10% nucleotide divergence in the capsid gene L1 and are numbered in chronological order from their isolation. More than 40 HPV types are known to infect the uterine cervix. Based on the induced benign, premalignant or malignant lesions, HPV is divided into low-risk ("LR", e.g. HPV types 6, 11, 42, 43 and 44) and high-risk types ("HR"). So far, thirteen HPV types are said to be HR HPV, namely HPV 16, 18, 31, 33, 35, 39, 45, 51, 52, 56, 58, 59 and 68. Those HR HPV, which have the highest prevalence, are HPV types 16, 18, 31, 33, 45 and 58 (de Villiers EM, et al., Int J Cancer. 2004 Mar 20;109(2):253-8). The high-risk types account for more than 99% of all invasive cervical cancers. Consequently, detection and identification of HPV types is very important. The high-risk types are by definition consistently found in high grade SIL (Squamous Intraepithelial Lesion) and carcinoma in situ, whereas low risk types are mainly found in low grade SIL. This epidemiological observation is supported by molecular findings. For instance, the E6 and E7 proteins from low-risk types 6 and 11 bind to cellular tumour suppressor proteins p53 and pRB too weakly to immortalize keratinocytes in vitro or to induce malignant transformation in vivo (Woodworth CD., et al., J. Virol. 1990;64:4767-4775). The circular dsDNA genome of low-risk HPV types remains episomal whereas the genome of high-risk HPV types is able to integrate into the human genome, causing genomic instability and thus propensity to tumour formation.

Therefore, early and precise recognition of these high-risk HPV types is the key factor for recovery from cervical cancer and for the appropriate diagnosis of other HPV-related malignancies, enabling their most adequate treatment.

Diagnosis of HPV by culture is not possible. Also diagnosis by detection of HPV antibodies appears to be hampered by insufficient sensitivity and specificity. Therefore, direct methods to diagnose an HPV infection are mainly based on detection of the viral DNA genome by different formats of DNA/DNA or RNA/DNA hybridization with or without prior amplification of HPV DNA. The polymerase chain reaction (PCR) is a method that is highly efficient for amplification of minute amounts of target DNA. Several different primer sets currently exist and are used for universal amplification in multiplex PCR setting of HPV DNA ("broad spectrum primers"). Three of these primer pairs, MY11/MY09, GP5/GP6 and the SPF system, are directed to conserved regions among different HPV types in the L1 region (Manos et al., Cancer Cells 1989, 7:209-214; Van den Brule et al., J Clin Microbiol. 1990 28(12):2739-43, WO9914377). The PGMY system, a modification of the MY09/11 is also used (Gravitt, P., 2000. J. Clin. Microbiol. 38:357-361). Another primer set, CPI/CPIIg, is directed to conserved regions in the El region (Tieben LM, et al., J Virol Methods. 1993;42:265-79). WO2006077102 discloses a further set of suitable primers.

For the identification of various HPV types, there are also several alternative methods. For example, HPV DNA can be directly typed by PCR primers that recognize only one specific type. This method is known as type-specific PCR. Such methods have been described for HPV types 6, 11, 16, 18, 31 and 33 The primers are aimed at the E5, L1, E6, L1, E2 and E1 regions of the HPV genome for types 6, 11, 16, 18, 31 and 33, respectively.

Another approach is performing general PCR amplification of a genomic part common for all HPV types, using e.g. broad spectrum primer sets, which is then followed by identification, or genotyping, by the obtained PCR products. At present, the most commonly employed method of genotyping involves hybridisation of the PCR products with type-specific probes or two cocktails of probes differentiating between the oncogenic and non-oncogenic HPV types, respectively. A similar typing method has been described without prior amplification of HPV DNA. In the hybrid capture assay (digene HPV hc2 Test; Qiagen), each sample is tested for a group of "high-risk" HPV types (i.e., 16, 18, 31, 33, 35, 39, 45, 51, 52, 56, 58, 59 and 68) and for another group of "low-risk" HPV types (i.e., 6, 11, 42, 43 and 44) (Cox et al., Am J Obstet. Gynecol. 1995; 172(3):946-54).

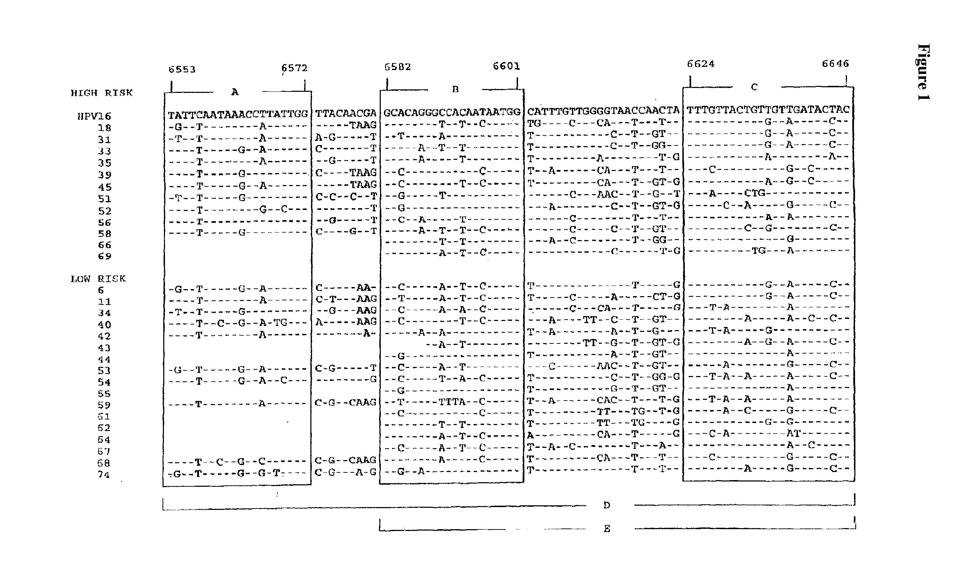

The HPV SPF detection technology ("SPF" standing for "short PCR fragment") and its suitable HPV typing system are disclosed in WO9914377. The method first utilises a broad-spectrum PCR amplification of a conserved 65 bp region of the HPV L1 gene (Figure 1), and then uses a reverse line blot hybridization with type specific probes for HPV genotyping.

The SPF primers set is characterised by a higher analytical sensitivity compared to GP5+/6+ (150bp) and (PG)MY09/11 (450bp) consensus primers (Kleter B, et al., J Clin Microbiol 1999;37:2508-17; Perrons C, et al., J Clin Virol. 2005: 32; 278-285), because of its shorter amplification product (65bp). In addition, it is predicted to have the capability of amplifying at least 54 HPV types. Therefore, it provides an excellent tool for HPV detection in large-scaled trials, especially for the analysis of highly degraded DNA, such as DNA from formalin-fixed paraffin-embedded tissue, and for the detection of HPV DNA in self-collected samples such as urine specimens (Payan C, et al., J Clin Microbiol. 2007: 45; 897-901). The assays based on the SPF amplification technology followed by a reverse line probe-based genotyping (LiPA assay, Innogenetics, Belgium) show a good clinical performance compared to other commercial tests for HPV genotyping in cervical cell specimens and formalin-fixed material (Galan-Sanchez F, et al., Acta Cytol 2011;55,341-3; Martró E ,et al., Enferm Infecc Microbiol Clin 2012;30,225-9) and are in addition commonly used in epidemiological studies and vaccination trials (Castellsagué X, et al., J Med Virol. 2012;84,947-956; Kovanda A, et al., Acta Dermatovenerol Alp Panonica Adriat. 2009;18,47-52).

However, even with such advanced technology, sensitive and exact diagnosis of HPV infection still remains a challenge. Firstly, new HPV genotypes are being constantly discovered, linked to human cancerogenesis or emerging as more noxious than previously estimated. An efficient detection method should be capable of detecting all of them with comparable sensitivity in order not to miss the more noxious and cancerogenic genotypes in cases of patients infected with multiple HPV types. However, in multiplex-PCR-based applications, such as SPF technology, that use a mix of multiple primers for detection of many slightly varying products, there exists a possibility of a bias in amplification of some products over others. This creates a challenge to develop a balanced combination of primers that would allow for a uniform and equally-efficient amplification of all clinically relevant genotypes.

But even after designing such difficult to obtain combination of primers, HPV genotyping is still associated with substantial workload, time and costs, which largely stem from the fact of relatively low prevalence of HPV infection in population. It is estimated that only 5 to 15% of the screening population (i.e. women aged 25-64 years) is HPV-positive (Cuzick et al., 2008 Cuzick J,et al., Vaccine 2008;26 Suppl 10:K29-41), therefore many of the samples that undergo testing and typing for HPV will not even contain the pathogen, causing waste of costly reagents and unnecessarily increased workload.

The present invention addresses such a need for improvements by providing a combination of SPF primers for uniform, sensitive and unbiased PCR amplification of a broad selection of HPV genotypes, including all high-risk (HR) ones. The present invention further provides a diagnostic kit and proposes a method based on said combination of SPF primers, for amplification and detection of a broad selection of HPV genotypes, including all high-risk (HR) ones, and allowing easy and economical identification thereof.

These and other advantages of said invention are presented in further- listed embodiments.

### Aim of the invention

The aim of this invention is to develop an optimised combination of SPF primers in order to reach the same unbiased detection level and analytical sensitivity for a broad selection of HPV genotypes, including all high-risk (HR) ones, in a multiplex PCR.

More particularly, the aim of this invention is to provide a combination of SPF primers that enables efficient amplification simultaneous with detection of a broad selection of HPV genotypes, including all high-risk (HR) ones, in real-time (RT) PCR, thus allowing rapid selection of HPV-positive samples prior to subjecting them to costly genotyping procedures.

Importantly, said combination of SPF primers have been developed to ensure unbiased and equal PCR amplification for a broad selection of HPV genotypes, including all high-risk (HR) ones, thus allowing detection and subsequent identification of diagnostically most relevant HPV genotypes, even in samples co-infected by multiple types of HPV.

The further aim of present invention is to compose diagnostic kits comprising said combination of SPF primers, permitting rapid and reliable detection of a broad selection of HPV genotypes, including all high-risk (HR) ones, and possibly their further identification.

Finally, it is also an aim of the present invention to provide cost-efficient and sensitive method of HPV detection based on said optimised SPF primers combination. In a preferred embodiment the method involves simultaneous amplification and detection of HPV DNA in a biological sample using RT-PCR or a quantitative RT-PCR, thus allowing selection of only samples infected with HPV for further genotyping. The ultimate aim of developing such combination of primers and diagnostic method is to ensure robust identification of clinically relevant viral genotypes and at the same time reduce costs and workload by eliminating genotyping of negative samples.

All the aims of the present invention are met by the following specific embodiments.

### Brief description of the figures and tables

Figure 1. Design principles of SPF primers (regions A, B, and C) and probes for hybridisation (regions D and E). Alignment of HPV sequences from WO9914377.
Table 1. DNA sequences of 11 SPF PCR primers forming the minimal combination for unbiased detection of 13 HR HPV genotypes ("SPF11").
Table 2. Sequences of additional SPF primers for obtaining a PCR primer combination for broader and unbiased detection of HPV genotypes ("SPF11+ ").
Table 3. Sequences of a primer pair for amplification a genomic fragment of human HLA-DPB1 gene, suitable for a control or reference amplicon in PCRs using SPF primers.
Table 4. Overview of sensitivity of old ("SPF10") VS new ("SPF11") VS new extended ("SPF11+") combination of SPF primers towards different HR HPV genotypes.
Table 5. Comparison of the genotyping results of 27 cervical samples obtained with the SPF11 conventional PCR and real-time PCR in combination with the LiPA assay (Cp, crossing point; Tm, melting temperature; HPV69/71, HPV types 69 and 71 are identified by a single probe on the LiPA and cannot be distinguished; HPVX, untypeable HPV-positive sample; neg, negative).
Table 6. Detection of HPV 16 and HPV 31 genotypes in mixtures containing relative amounts of HPV 16 and HPV 31 full-length plasmids ranging from 1:1 to 10³:1.

### Detailed description of the invention

The present invention provides a combination of SPF PCR primers for the amplification of HPV-DNA and detection of HPV possibly present in a biological sample, allowing efficient and unbiased detection with high analytical sensitivity for a broad selection of HPV genotypes, including all high-risk (HR) ones, in a multiplex PCR reaction.

According to one preferred embodiment of the present invention provides a minimal combination of 11 SPF primers allowing for unbiased and equal PCR amplification of all 13 HR HPV genotypes, being the genotypes of HPV16, 18, 31, 33, 35, 39, 45, 51, 52, 56, 58, 59 and 68.

The choice of the minimal 11 SPF primer combination, further referred to as "SPF11", is substantiated in Example 1. The SPF11 sequences are listed in the Table 1.

In another embodiment, the invention provides a combination of the minimal 11 SPF primers in addition comprising at least one additional SPF primer. Such combination is then referred to as "SPF11+" or "SPFn" where "n" is the number of primers in said combination, and is designed to ensure unbiased and sensitive amplification in a multiplex PCR for a broad selection of HPV genotypes, including all high-risk (HR) ones. In a preferred embodiment, said at least one additional SPF primer is selected from a group of sequences listed in Table 2. An example of such "SPF11+" PCR primer combination for broader and unbiased detection of HPV genotypes is outlined in Example 1.

In an alternative embodiment, at least one of the SPF primers forming the PCR primer combination for the unbiased amplification and detection of HPV in a biological sample, is conjugated at its 5' end with a known in the art moiety, such as biotin, that can enable HPV genotype identification, using the PCR product obtained with such modified at least one SPF primer, according to any of the nucleic acid identification methods known in the art. Examples of combinations of such biotin-modified SPF primers can be found in Examples 1, 2, and 3.

In a further preferred embodiment of the present invention, any of the afore-described combinations of PCR primers for sensitive and unbiased detection of a broad selection of HPV genotypes, including all high-risk (HR) ones, form part of a diagnostic kit for the detection and optionally identification of HPV in a biological sample. According to one preferred embodiment, said diagnostic kit, in addition to any of the afore-described combination of PCR primers, comprises a plurality of reagents required for carrying out DNA amplification by PCR, including DNA polymerase, substrates such as dNTPs, and appropriate reaction solutions such as buffers. In a preferred embodiment, said plurality of reagents would also include means for performing HPV detection by real-time (RT)-PCR, such as a solution of a dsDNA-specific fluorescent dye, such as SYBR Green.

In a particular embodiment, said diagnostic kit could further comprise means for identification of at least one HPV type, such as beads or strips with at least one HPV-type specific probe capable of specific hybridization with at least one PCR product obtained using any of the afore-described combinations of SPF PCR primers. HPV genotype identification using such probes is known in the art and examples of identification of PCR products obtained using a combination of SPF PCR primers according to any of the embodiments described herein is provided in Examples 1, 2, and 3.

In a preferred embodiment such diagnostic kit would also provide at least one set of primers for the amplification of human genomic DNA, said amplification serving as an internal PCR validation control and/or control for normalisation of the amplified HPV DNA obtained using a combination of SPF PCR primers according to any of the embodiments described herein. An example of such pair of primers targeting an 80 bp fragment from the human HLA-DPB1 gene is provided in the Table 3.

According to a further aspect, the present invention provides a method of unbiased amplification of a broad selection of HPV genotypes, including all high-risk (HR) ones, ensured by the use of any of the described herein combinations of SPF PCR primers, thus allowing sensitive and efficient detection of HPV possibly present in a biological sample, even in cases of multiple HPV infections, as demonstrated in Example 3.

According to a preferred embodiment, said method not only would ensure a robust identification of clinically relevant HPV genotypes but also allow selection of HPV-infected samples before subjecting them to more costly and time-consuming HPV type identification procedures, such as hybridisation to probes or direct DNA sequencing. In such embodiment, selection of samples for HPV genotyping would depend on the positive detection of HPV PCR product generated in the amplification step, said detection being possible by gel-analysis of said PCR products, or, preferably, being real-time (RT) detection in a real-time PCR using for example a fluorescent dye, such a SYBR Green, or fluorescent probes, such as Taqman probes. An example of SPF11-based RT-PCR, followed by genotyping of HPV-positive samples, using LiPA probe-based technology, is provided as Example 2. The initial selection of HPV-positive samples, using a robust and unbiased combination of primers, as described herein, prior to HPV-type identification procedure, allows to significantly reduce costs and workload associated with genotyping of negative samples.

In a preferred embodiment of above-described method, the amplification and detection are performed simultaneously by means of RT-PCR in any setup known in the art, including quantitative RT-PCR allowing assessment of the viral load in the infected sample.

According to another preferred embodiment, the method provided by the present invention in addition comprises a step of identification of the amplified HPV-DNA detected in a previous step. Such identification of a genotype can be performed according to any nucleic sequence identification method known in the art. In one possible embodiment, HPV DNA is identified using DNA sequencing. Many techniques for DNA sequencing are currently available and genotyping by sequencing (GBS) using next-generation sequencing technologies is becoming increasingly important as a cost-effective and unique tool. One possible and known in the art embodiment includes solid-phase sequencing of biotinylated PCR products with streptavidin-coated magnetic beads (Perry DJ., Methods Mol Med. 1999;31:49-54).

In an alternative embodiment, amplified HPV DNA can be genotyped based on its melting curve analysis. In this type of analysis dissociation-characteristics, such as melting point, of the amplified HPV-DNA fragments are assessed during heating. Currently, there exist several known in the art approaches to measure DNA strand dissociation, from the oldest ones using UV absorbance, to more recent and currently common techniques based on fluorescence.

In a yet another embodiment of the present invention, HPV genotype identification is performed based on specific hybridisation of the PCR-amplified HPV DNA fragments to HPV-type specific nucleic acid probes. Many examples of such probes are known in the art. Usually, they are attached by means known in the art to a solid support such as a bead or a strip, for example LiPA strips with probes compatible with the SPF PCR products and disclosed in WO9914377. These probes are designed to specifically hybridize to the D region, or more particularly to a 22 bp region situated between the B region and the C region, of only one HPV genome, as indicated in Figure 1.

In a final embodiment, the present invention provides the sequences of primers that combined together allow for a broad selection of HPV genotypes, including all high-risk (HR) ones.

To a person skilled in the art it will be obvious that other primers may be used or developed in order to amplify fragments within or overlapping with said D region, shown in Figure 1. The preferred primers are listed in Table 1 and 2, and were designed using novel computational approaches to form combinations that allow efficient and unbiased detection with high analytical sensitivity for a broad selection of HPV genotypes. The minimal SPF11 combination ensure unbiased and equal PCR amplification of all 13 HR HPV genotypes, however alternative combinations of primers listed in Table 1 and 2 are possible to selectively amplify alternative groups of HPV types. Therefore, present invention provides said primer sequences as novel building blocks for forming such diagnostic combinations.

### Definitions

As used herein, the term "SPF primer" shall refer to any of the primers designed according to the rules disclosed in WO9914377. Briefly, as used herein "SPF primer" shall refer to any of the primers designed to amplify a short PCR fragment ("SPF") of about 65 nucleotides from a highly variable region of HPV L1 locus. The short region of the L1 gene is shown in Figure 1.

"SPF primers" are further characterised in that:
- a 5'-primer (or "forward" SPF primer) specifically hybridises to the B region of the genome of at least one HPV type, said B region being indicated in Figure 1, and,
- a 3'-primer (or "reverse" SPF primer) specifically hybridises to the C region of the genome of at least one HPV type, said C region being indicated in Figure 1;
where said B region of the genome of at least one HPV type, is situated at position 6601 of the genome of HPV 16, or at the corresponding position of any other HPV genome, as indicated in Figure 1, and where said C region of the genome of at least one HPV type, is situated at position 6624 of the genome of HPV 16, or at the corresponding position of any other HPV genome, as indicated in Figure 1.

### Examples

### Example 1: Sensitivity of SPF11 and SPF11+ primer combinations.

The aim of this study was to develop a combination of SPF PCR primers capable of reach same sensitivity for at least all high-risk genotypes (and preferably also several low risk HPV genotypes)

Methods: Multiple HPV SPF primers were designed and tested for sensitivity of HPV detection. In addition to the HPV primers, PCR reaction control primers were designed to amplify a 80 bp fragment of human HLA DPB1 fragment (Table 3). The old SPF10 primer set was compared with all possible SPF11 combinations to investigate the sensitivity of the newly assembled set. The influence on the sensitivity by SPF11+ was tested by adding additional primers (Table 2) to the optimised SPF11 mix (Table 1). HPV plasmids containing the 65 nt fragment of HPV L1 gene (Figure 1) were used as target; dilutions were prepared in LiPA dilution medium containing 1 ng/µl human DNA. The primer set sensitivity was tested for all high-risk HPV genotypes (and all probably high-risk (pHR) HPV genotypes) for the dilution-range 3 - 30 - 300 - 3000 - 30.000 cp/PCR. The HPV typing was performed using probes on INNO-LiPA HPV Genotyping strips. The strips were scanned with the LiRAS scan software for quantification of the intensity of the color reaction (OD-measurement): a value greater than or equal to 0.01053 was considered as a positive reaction; a value less than 0.01053 was considered as a negative reaction.

Results: Improved sensitivity of SPF11 set over the previous primer set ("SPF10") at least for high risk (HR) HPV genotypes is shown in Table 4. Table 4 also shows improvement of detection of other HPV (potentially HP," pHR") types upon addition of more primers (here 5 shown) to the working set of 11 ("SPF11+5").

Conclusion: Analytical sensitivity between 30 and 300 copies of HPV plasmid target per PCR was obtained for all (probably) high risk HPV genotypes. Therefore, the new combination of at least 11 SPF PCR primers ("SPF11+") allows unbiased and efficient detection of a broad selection of HPV genotypes, including all high-risk (HR) ones.

### Example 2: SPF11 RT-PCR using clinical samples

The aim of this study was to evaluate the clinical performance of the SPF11 real-time PCR. Briefly, cervical samples were subjected to the SPF conventional PCR in combination with the LiPA assay for HPV detection and typing. Subsequently, the SPF real-time PCR was performed to enable comparison between the SPF conventional and real-time PCR results

**Population study:** The cervical samples for HPV DNA analysis were obtained from women attending the Obstetrics Gynecology Unit at the University Hospital of Saint-Etienne (France). The cervical cells were collected with a cytobrush and transferred into PreservCyt medium (Hologic Inc., Marlborough, MA, USA). DNA extraction was performed on a sample volume of 200 µl by the NucliSENS easyMAG system on the EasyMag Extraction Platform (bioMérieux, Marcy l'Etoile, France)

**SPF real-time PCR and LiPA assay:** Thirty-nine cervical samples were subjected to the SPF11 real-time PCR. In brief, SPF real-time PCR was performed in a final reaction volume of 50µl containing 10µl SYBR green I (1/20 000 final dilution) (Sigma, Saint Louis, MO, USA), 1x PCR buffer II (Applied Biosystems, Foster City, CA, USA), 4mM MgCl2 (Applied Biosystems), 200µM of each deoxynucleoside triphosphate (Roche Applied Science), 300nM of each of the biotinylated forward and reverse SPF11 primers (Innogenetics), 1.5U of AmpliTaq Gold® (Applied Biosystems) and 10µl template. The amplification conditions were 10 min at 95°C for AmpliTaq Gold activation, followed by 40 cycles of 30 s at 95°C for denaturation, 45 s at 52°C for annealing and 45 s at 72°C for extension. The melting program was 5 s at 95°C followed by an increase of 2.2°C/s from 52°C up to 97°C. Samples with a melting temperature (Tm) in the range of 78-84°C were considered HPV-positive. Samples without a sharp peak on the melting curves or with weak peaks (width: 0, height: 0), or with a Tm outside the predefined range were considered as HPV-negative. Subsequently, the HPV-positive samples were genotyped using the LiPA assay at an increased hybridisation temperature and with a lower amplicon volume (1µl)

**Results:** Thirty-nine cervical samples, of which the HPV-status was determined with the conventional SPF PCR-LiPA, were subjected to the SPF real-time PCR. The SPF real-time PCR identified 27 HPV-positive samples and 12 HPV-negative samples. All samples that were HPV-positive by the conventional protocol, were also positive for HPV infection by real-time PCR-positive. The observed percentage of agreement between both assays was 92.3% (κ = 0.831).

The LiPA assay was carried out on the 27 real-time PCR-positive samples to determine the HPV genotype. Table 5 shows the genotyping results for both assays. Of the 20 concordant samples, ten contained a single genotype, while the other ten displayed multiple genotypes. Six samples showed double infections and four showed at least triple infections. The three compatible samples showed multiple infections. The LiPA assay in combination with the SFP real-time PCR allowed to detect an additional type in two samples. In the two samples with discordance, the SPF11 real-time PCR resulted in the detection of an HPV58 and an untypeable HPV type (HPVX).

When analysing the multiple infections, both assays showed a very good agreement for the number of HPV types present (κ = 0.834). Importantly, real-time PCR occurred more efficient in HPV detection of multiple infections as higher number of multiple infections were identified by the SPF real-time PCR (n=13) compared to the conventional PCR (n = 11).

**Conclusions:** The aim of this study was to evaluate the clinical performance of the SPF11 real-time PCR through comparison with the conventional SPF11 results of 39 cervical samples. Overall, the results demonstrate a very good strength of agreement between both assays for individual HR-, probable HR- and LR-HPV genotype detection

SPF real-time PCR allows the simultaneous amplification and detection of HPV DNA in clinical samples. That way, LiPA or alternative genotyping analysis of the HPV-negative samples can be avoided, reducing workload and cost. Indeed, the SPF11 real-time PCR identified 12 of the 39 samples as HPV-negative. These samples should not be subjected to the LiPA assay, reducing workload and cost.

In addition, the results in Table 5 indicate that the SPF11 real-time PCR might detect more multiple HPV infections than the conventional PCR approach.

The real-time PCR can therefore be used for the detection and subsequent genotyping of HPV in cervical samples. As it immediately identifies the HPV-negative samples, less LiPA analyses are required, reducing the workload and costs.

### Example 3: Detection of multiple infections using SPF11 RT-PCR

**Experimental setup:** SYBR Green-based real-time PCR protocol for the SPF11 primers was conducted on LightCycler^{®} 480 (Roche). The detection of two genotypes in a single sample was evaluated to assess the extent of competition for SPF11 primers and PCR reagents. DNA plasmids of full-length HPV 16 and HPV 31 were serially diluted (10-fold dilution range: 10⁵ to 10² copies/µl) and mixed in relative amounts ranging from 1:1 to 10³:1. The SPF11 real-time PCR was carried out, followed by the LiPA assay.

**Results:** The competition for SPF10 primers and PCR reagents was evaluated using HPV 16 and HPV 31 plasmid mixtures (1:1 to 10³:1). The results are shown in Table 6. HPV 16 and HPV 31 could be detected by the LiPA assay in samples containing up to a 1000-fold more HPV 31 than HPV 16. Similarly, when a fixed amount of HPV 31 was mixed with an increasing amount of HPV 16, the LiPA was able to identify both genotypes in all plasmid mixtures (1:1 to 10³:1).

**Conclusions:** SPF11 real-time PCR is capable of detecting multiple infections in a biological sample, as indicated by the genotyping of plasmid mixtures containing up to a 1,000-fold more HPV 16 than HPV 31 or vice versa. This demonstrates excellent analytical sensitivity and specificity of the SPF11+ real-time PCR. Melting peak analysis allows identification of the specific HPV amplicons. The HPV-positivity of the sample can therefore be determined before subjecting it to the LiPA assay.

**Table 1**

| Forward/Reversed | Name | Sequence (5' -> 3') |
|---|---|---|
| Forward Primer | HPV-X pri SPF1A | GCICAGGGICACAATAATGG |
| Forward Primer | HPV-X pri SPF1 B | GCICAGGGICATAACAATGG |
| Forward Primer | HPV-X pri SPF1C | GCICAGGGICATAATAATGG |
| Forward Primer | HPV-X pri SPF1 D | GCICAAGGICATAATAATGG |
| Forward Primer | HPV-X pri SPF1 H | GCTCAGGGTTTAAACAATGG |
| Reverse Primer | HPV-X pri SPF2D | GTIGTATCIACTACAGTAACAAA |
| Reverse Primer | HPV-X pri SPF2J | GTGGTATCCACAACIGTGACAAA |
| Reverse Primer | HPV-X pri SPF2K | GTAGTITCCACAACAGTAAGAAA |
| Reverse Primer | HPV-X pri SPF2M | GTIGTATCTACAACIGTTAAAAA |
| Reverse Primer | HPV-X pri SPF2P | GTAGTATCAACACAGGTAATAAA |
| Reverse Primer | HPV-X pri SPF2B | GTIGTATCIACAACAGTAACAAA |

**Table 2**

| Forward/Reversed | Name | Sequence (5' -> 3') |
|---|---|---|
| Forward Primer | HPV-XpriSPF1E | GCICAGGGICACAACAATGG |
| Forward Primer | HPV-XpriSPF1G | GCICAGGGICAAAATAATGG |
| Reverse Primer | HPV-XpriSPF2Z | GTAGTGTCCACCACAGTAATAAA |
| Reverse Primer | HPV-XpriSPF2R | GTGGTATCAACIACGGTAACAAA |
| Reverse Primer | HPV-XpriSPF2W | GTAGTGTCAACACAAGTAAIAAA |
| Reverse Primer | HPV-XpriSPF2Y | GTIGTATCCACAACIGTAAGAAA |
| Forward Primer | HPV-XpriSPF1F | GCICAAGGICATAACAATGG |
| Reverse Primer | HPV-XpriSPF2Q | GTGGTGTCIACCACAGTAACAAA |
| Reverse Primer | HPV-XpriSPF2U | GTGGTATCCACAACAGTTACAAA |
| Reverse Primer | HPV-XpriSPF2V | GTGGTGTCAACACAIGTAAIAAA |

**Table 3**

| Forward/Reversed | Name | Sequence (5' -> 3') |
|---|---|---|
| Forward Primer | HLA pri DPB1 | TTCGACAGCGACGTGGG |
| Reverse Primer | HLA pri DPB2 | GTCCTTCTGGCTGTTCCAG |

**Table 4**

| HPV GT | Risk | SPF10 | SPF11 | SPF11+5 |
|---|---|---|---|---|
| 16 | HR | 30 | 30 | 30 |
| 18 | HR | 30 | 30 | 30 |
| 31 | HR | 30 | 30 | 30 |
| 33 | HR | 30 | 30 | 30 |
| 35 | HR | 30 | 30 | 30 |
| 39 | HR | 30 | 30 | 30 |
| 45 | HR | 300 | 30 | 30 |
| 51 | HR | 30 | 30 | 30 |
| 52 | HR | 30 | 30 | 30 |
| 56 | HR | 30 | 30 | 30 |
| 58 | HR | 3000 | 30 | 30 |
| 59 | HR | 30000 | 30 | 30 |
| 68 | HR | >30000 | 300 | 30 |
| 26 | pHR | 3 | 30 | 30 |
| 53 | pHR | 30 | 300 | 30 |
| 66 | pHR | 300 | 300 | 30 |
| 70 | pHR | 300 | 30 | 30 |
| 73 | pHR | 30000 | 300 | 30 |

**Table 5**

| No Sample | SPF11 PCR assay | | Cp | Tm (°C) |
|---|---|---|---|---|
| | conventional | real-time | | |
| | HPV type | HPV type | | |
| 1 | 31 | 31 | 19.2 | 80.6 |
| 2 | 52 | 52 | 19.9 | 83.4 |
| 3 | 70 | 70, 82 | 27.8 | 81.6 |
| 4 | 6,33,68 | 6,33,68 | 18.3 | 80.5 |
| 5 | 73 | 73 | 29.7 | 79.4 |
| 6 | 58 | 58 | 12.2 | 82.5 |
| 7 | 52 | 52 | 20.4 | 83.7 |
| 8 | 31 | 31 | 33.7 | 81.5 |
| 9 | 53, 73 | 53, 73 | 28.7 | 78.4, 83.3 |
| 10 | 51 | 51 | 23.3 | 82.3 |
| 11 | 35, 53, 54 | 35, 53, 54 | 25.3 | 80.4 |
| 12 | 82 | 82 | 37.6 | 82.7 |
| 13 | 51 | 51 | 28.4 | 82.2 |
| 14 | 16,82 | 16,82 | 33 | 82.2 |
| 15 | 70, 74 | 70, 74 | 26.1 | 81.1 |
| 16 | 51,70 | 51,70 | 21.6 | 82.2 |
| 17 | 39 | 39, 54 | 22.6 | 80.4 |
| 18 | 16,74 | 16,74 | 35.6 | 81.9 |
| 19 | 51,52 | 51,52 | 17.8 | 84.1 |
| 20 | 18,53, 69/71 | 18,53, 69/71 | 23.5 | 82.8 |
| 21 | 16,58 | 16,58 | 31.1 | 82.9 |
| 22 | 53 | 53 | 22.8 | 82.5 |
| 23 | 18,44,52 | 18,44,52 | 18 | 80.3 |
| 24 | 52 | 58 | 21.8 | 83.7 |
| 25 | neg | neg | 33.9 | 80.7, 84.1 |
| 26 | neg | neg | 36.3 | 81.9 |
| 27 | neg | HPVX | 36.8 | 80.8 |

**Table 6**

| HPV type in sample | HPV type ratio | SPF10 real-time PCR | | HPV type by LiPA |
|---|---|---|---|---|
| | | Cp^{a} | Tm(°C)^{b} | |
| 16 | 1 | 33.56 | 81.5 | 16 |
| 31 and 16 | 1:1 | 30.47 | 80.8 | 31+16 |
| 31 and 16 | 10:1 | 28.71 | 80.7 | 31+16 |
| 31 and 16 | 10²:1 | 24.68 | 80.8 | 31+16 |
| 31 and 16 | 10³:1 | 19.5 | 80.9 | 31 |
| 31 | 1 | 30.6 | 80.9 | 31 |
| 16 and 31 | 1:1 | 30.52 | 80.9 | 16+31 |
| 16 and 31 | 10:1 | 29.32 | 81.1 | 16+31 |
| 16 and 31 | 10²:1 | 26.91 | 81.5 | 16+31 |
| 16 and 31 | 10³:1 | 21.92 | 81.1 | 16+31 |

**Table 1**

| Forward/Reversed | Name | Sequence (5' -> 3') |
|---|---|---|
| Forward Primer | HPV-X pri SPF1A | GCICAGGGICACAATAATGG |
| Forward Primer | HPV-X pri SPF1 B | GCICAGGGICATAACAATGG |
| Forward Primer | HPV-X pri SPF1C | GCICAGGGICATAATAATGG |
| Forward Primer | HPV-X pri SPF1 D | GCICAAGGICATAATAATGG |
| Forward Primer | HPV-X pri SPF1 H | GCTCAGGGTTTAAACAATGG |
| Reverse Primer | HPV-X pri SPF2D | GTIGTATCIACTACAGTAACAAA |
| Reverse Primer | HPV-X pri SPF2J | GTGGTATCCACAACIGTGACAAA |
| Reverse Primer | HPV-X pri SPF2K | GTAGTITCCACAACAGTAAGAAA |
| Reverse Primer | HPV-X pri SPF2M | GTIGTATCTACAACIGTTAAAAA |
| Reverse Primer | HPV-X pri SPF2P | GTAGTATCAACACAGGTAATAAA |
| Reverse Primer | HPV-X pri SPF2B | GTIGTATCIACAACAGTAACAAA |

**Table 2**

| Forward/Reversed | Name | Sequence (5' -> 3') |
|---|---|---|
| Forward Primer | HPV-XpriSPF1E | GCICAGGGICACAACAATGG |
| Forward Primer | HPV-XpriSPF1G | GCICAGGGICAAAATAATGG |
| Reverse Primer | HPV-XpriSPF2Z | GTAGTGTCCACCACAGTAATAAA |
| Reverse Primer | HPV-XpriSPF2R | GTGGTATCAACIACGGTAACAAA |
| Reverse Primer | HPV-XpriSPF2W | GTAGTGTCAACACAAGTAAIAAA |
| Reverse Primer | HPV-XpriSPF2Y | GTIGTATCCACAACIGTAAGAAA |
| Forward Primer | HPV-XpriSPF1F | GCICAAGGICATAACAATGG |
| Reverse Primer | HPV-XpriSPF2Q | GTGGTGTCIACCACAGTAACAAA |
| Reverse Primer | HPV-XpriSPF2U | GTGGTATCCACAACAGTTACAAA |
| Reverse Primer | HPV-XpriSPF2V | GTGGTGTCAACACAIGTAAIAAA |

**Table 3**

| Forward/Reversed | Name | Sequence (5' -> 3') |
|---|---|---|
| Forward Primer | HLA pri DPB1 | TTCGACAGCGACGTGGG |
| Reverse Primer | HLA pri DPB2 | GTCCTTCTGGCTGTTCCAG |

## Claims

1. A combination of PCR primers for the amplification of HPV-DNA and detection of HPV in a biological sample **characterised in that** said combination comprises at least the 11 SPF primers listed in Table 1.

2. A combination of PCR primers according to claim 1, further comprising at least one additional SPF primer.

3. A combination of PCR primers according to claim 2 wherein said at least one additional SPF primer is selected from the ones listed in Table 2.

4. A combination of PCR primers according to any of the previous claims, wherein at least one of said primers is conjugated with a moiety, such as biotin, at its 5' end, that enables identification of a PCR product obtained using said combination of SPF primers, according to any of the nucleic acid identification methods known in the art.

5. A combination of PCR primers according to any of the preceding claims, which is part of a diagnostic kit for the amplification of HPV-DNA and detection and optionally identification of HPV in a biological sample.

6. A diagnostic kit for the amplification, detection and optionally identification of HPV-DNA in a biological sample, said diagnostic kit comprising:
(a) the combination of PCR primers according to claim 5 , and
(b) one or more reagents required for carrying out DNA amplification by PCR, preferably by real-time (RT)-PCR, most preferably RT-PCR using dsDNA-specific fluorescent dye, such as SYBR Green.

7. A diagnostic kit according to the preceding claim, comprising (c) means for identification of at least one HPV type, such as at least one HPV-type specific probe capable of specific hybridization with at least one HPV-DNA fragment amplified using the combination of PCR primers according to claim 5.

8. A diagnostic kit according to claim 6 or 7, further comprising (d) a primer pair suitable for amplifying a genomic fragment of 65 bp from human HLA-DPB1 gene, wherein said fragment can be used during or after amplification as a control or reference sequence, and wherein said primer pair is listed in Table 3.

9. A method of detection of HPV in a biological sample by PCR, said method comprising:
(a) amplification of HPV-DNA by PCR, preferably real-time (RT)-PCR
(b) detection of the thus amplified HPV-DNA,
**characterised in that** the amplification step is performed using the combination of PCR primers according to any of claims 1-5.

10. A method according to claim 9 wherein said PCR is real time (RT)-PCR or a quantitative RT-PCR (RT-qPCR).

11. A method according to claims 9 or 10, further comprising a step (c) of identification of the amplified HPV-DNA detected in step (b).

12. A method according to claim 11 wherein identification of at least one HPV type present in the biological sample is performed using DNA sequencing of the detected HPV-DNA.

13. A method according to claim 11 wherein identification of at least one HPV type present in the biological sample is deduced based on the melting curve analysis of the detected HPV-DNA.

14. A method according to claim 11 wherein identification of at least one HPV type present in the biological sample is based on contacting the detected HPV-DNA with at least one probe capable of specific hybridization to any of said PCR products.

15. A primer pair suitable for amplifying a genomic fragment of 80 bp from human HLA-DPB1 gene, wherein said fragment can be used during or after amplification as a control or reference sequence in a method according to any of claims 8-10, and wherein said primer pair is listed in Table 3.

16. Any of the PCR primers from the combination of PCR primers according to claims 1 and 3, selected from the group consisting of the sequences listed in Tables 1 and 2.
